# EUROPÄISCHE PATENTANMELDUNG

(11) **EP 2 886 337 A1**
(43) Veröffentlichungstag der Anmeldung: **24.06.2015**
(21) Anmeldenummer: 15150168.1
(22) Anmeldetag: 14.04.2010
(51) Int. Cl.: B32B 25/08, B64C 39/00, B32B 25/10

(54) **Verbundbauteile aus wärmehärtenden Harzen und Elastomeren**

(30) Priorität: 14.04.2009 DE 202009005438 U; 14.05.2009 DE 202009006966 U
(62) Teilanmeldung aus: 10713853.9
(71) Anmelder: Gummiwerk Kraiburg GmbH & Co. KG, 84478 Waldkraiburg (DE)
(72) Erfinder: Zahn, Judith, 84478 Waldkraiburg (DE); Plenk, Florian, 84478 Waldkraiburg (DE); Schaube, Jens, 84478 Waldkraiburg (DE)
(74) Vertreter: Wiese, Gerhard

(57) **Zusammenfassung**

Die Erfindung betrifft ein Kunststoff-Verbundbauteil (10), das von einer dünnen harten Kunststoff-Außenschicht (12), wenigstens einer daran nach innen anschließenden Elastomerschicht (14) und wenigstens einer daran nach innen anschließenden Metall- und/oder Kunststoff-Trägerschicht (16) aus einem Faserverstärkten Kunststoff (FVK), einem Carbonfaserverstärkten Kunststoff (CFK) oder einem Glasfaserverstärkten Kunststoff (GFK) gebildet wird und als Aufprall-Schutzteil, als Splitter-Schutzteil, als Schutzteil gegen ein plötzliches Totalversagen oder als Schutzteil gegen Vibrationen und Vibrationsschäden, gegen Resonanzen, zur Schwingungsdämpfung oder zur akustischen Dämpfung an einem Bauteil angeordnet ist oder dieses zumindest teilweise bildet.

## Beschreibung

Die Erfindung betrifft Verbundbauteile aus wärmehärtenden Harzen und Elastomeren gemäß dem Oberbegriff der unabhängigen Schutzansprüche.

Der derzeitige Stand der Technik zur Herstellung von Verbundbauteilen aus einem wärmehärtenden Harz mit einer Elastomerschicht besteht darin, dass zunächst ein Formteil aus wärmehärtendem Harz hergestellt und hierauf in einem erneuten Arbeitsgang die Elastomerschicht aufgebracht wird. Dieses System findet derzeit Anwendung bei Elastomer-beschichteten Walzen oder Zylindern sowie anderen Mehrkomponenten-Formteilen. Die Herstellung bzw. Aushärtung des Harz-basierenden Formteils erfolgt in Autoklaven oder Heizpressen bei erhöhter Temperatur, wobei zusätzlich eine verstärkende Einlage aus einem Gewebe oder faserartigen Materialien mit eingebracht sein kann. Bei beiden Vorgängen erfolgt die Aushärtung durch eine chemische Reaktion. Auf diese Weise können z. B. Rollen, Räder, rutschfeste Beschichtungen aus Kunststoff oder mit einer gewissen Eigensteifigkeit versehene Elastomer-Artikel hergestellt werden.

Bei der Verwendung von Kunstoffplatten z.B. im Fahrzeug- oder Schiffbau werden an den Kanten Elastomer-Profile befestigt z.B. aufgeklebt oder angeschraubt, um eine Abdichtung und den Ausgleich unterschiedlicher thermischer Ausdehnungskoeffizienten zu erreichen oder um Verspannungen und Quietschen bei durch die Fahrsituation bedingten elastischen Bewegungen der Fahrzeuge zu vermeiden.

Faserverstärkte Kunststoffe sind meist energieelastisch und spröde und können deshalb beim Auftreten eines Energieeintrags durch Schwingungen, Stöße, Schläge oder Beschuss wenig Energie aufnehmen bzw. absorbieren. Das kann zur Zerstörung des Bauteils führen, wobei dann scharfe und gezackte Bruchkanten auftreten, die ein Verletzungsrisiko darstellen. Gegebenenfalls müssen besondere Maßnahmen getroffen werden, um Energie zu absorbieren bzw. eine Zerstörung des Bauteils durch auftretende Resonanzen zu unterbinden. Faserverstärkte Kunststoffe sind auf Grund ihres Harzanteils leicht brennbar und führen im Fall eines Brandes dem Feuer zusätzlichen Brennstoff zu.

In der WO 2006/122749 A1 hat die Anmelderin bereits Verbundbauteile, Verfahren zu deren Herstellung und einige vorteilhafte Anwendungen dafür beschrieben.

Der vorliegenden Erfindung liegt die Aufgabe zugrunde, weitere vorteilhaft herstellbare Kunststoff-Verbundbauteile zu beschreiben, die ein verbessertes Crash-Verhalten aufweisen mit der Folge eines wirkungsvollen Splitterschutzes zur Vermeidung von Verletzungen und einer Vermeidung eines plötzlich auftretenden Totalversagens des Verbundbauteils.

Diese Aufgabe wird durch die in den verschiedenen unabhängigen Patentansprüchen angegebenen Kunststoff-Verbundbauteile gelöst. Vorteilhafte Ausgestaltungen der Erfindung sind den jeweiligen Unteransprüchen zu entnehmen.

Die Erfindung sieht vor, dass durch eine chemische Reaktion aus dem wärmehärtenden Harz eine harte Schicht als Bauteiloberfläche gebildet, gleichzeitig ebenfalls durch eine chemische Reaktion das Aushärten bzw. Vulkanisieren der mit einem Vernetzer versehenen Elastomerschicht erfolgt, der direkte Verbund der Elastomerschicht mit der harten Schicht erreicht und schließlich auch die Verbindung mit einer auf der Rückseite der Elastomerschicht angeordneten Kunststoff-Trägerschicht bewirkt wird.

Dabei kann in einer oder mehreren Schichten eine Gewebeeinlage, ein Fasermaterial oder Metallpulver enthalten sein.

Die Begriffe "Kunststoff-Außenschicht" und "nach innen anschließend" sind jeweils von der Nutzseite des Kunststoff-Verbundbauteils gesehen. Bei einem Innenverkleidungsteil eines Fahrzeugs ist die Kunststoff-Außenschicht dementsprechend die dem Fahrzeuginnenraum zugewandte Schicht.

Wenn bei der vorliegenden Erfindung von "Schichten" die Rede ist, können damit ganz oder auch nur partiell übereinander angeordnete oder ineinander eingebettete Streifen, Stücke oder Bereiche aus den genannten Materialien gemeint sein.

Von einem Kunststoff-Verbundbauteil im Sinne der vorliegenden Erfindung wird auch dann gesprochen, wenn eine oder mehrere Schichten des Kunststoff-Verbundbauteils aus Metall oder anderen, nicht als Kunststoffe zu bezeichnenden Materialien bestehen.

Die als Festigkeitsträger fungierende Kunststoff-Trägerschicht, die optional auch durch wenigstens eine metallische Trägerschicht ergänzt oder ersetzt werden kann, die darauf befindliche Elastomerschicht und die Kunststoff-Außenschicht werden in einem Arbeitsgang gemeinsam aufgebaut und anschließend unter Temperatureinwirkung im Autoklav oder einer Heizpresse gemeinsam ausgehärtet bzw. vulkanisiert. Alle beteiligten Rohmaterialien sind so aufeinander abgestimmt, dass sie unter gleichen Reaktionsbedingungen gleichzeitig ein chemisches Netzwerk aufbauen und zueinander eine Haftung ausbilden. Durch diese Vorgänge entsteht ein formstabiles Produkt. Die Aushärtetemperatur liegt vorzugsweise zwischen 80 und 200° C.

Der Aufbau des Mehrkomponentenproduktes aus wärmehärtendem Harz, der mit einem Vernetzer versehenen Elastomerschicht und der Metall- und/oder Kunststoff-Trägerschicht geschieht in mehreren Alternativen wie folgt:
- Gemäß einer ersten Alternative wird eine antihaftend beschichtete Form mit den verschiedenen Rohkomponenten der einzelnen Schichten bestückt und das Verbundteil unter Einwirkung von Druck und Temperatur ausgehärtet (Heißpressen).
- Gemäß einer zweiten Alternative wird das aus den Rohmaterialien vorkonfektionierte Produkt ohne Verwendung einer geschlossenen Form bei erhöhter Temperatur im Autoklav oder Heißluftofen ausgehärtet. Es kann dabei auf einem die Kunststoff-Trägerschicht bildenden Trägerkörper fixiert sein.
- Gemäß einer dritten Alternative wird das aus den Rohmaterialien vorkonfektionierte Produkt ohne Verwendung einer geschlossenen Form bei erhöhter Temperatur in einem Vakuumsack ausgehärtet.

Die antihaftende Beschichtung der Form kann erfolgen durch Paraffine, Silikon, Tenside, Fluorkohlenwasserstoffe (z.B. Teflon).

Als Kunstharze sind bevorzugt verwendbar: Polyesterharze, Phenol-FormaldehydHarze, Cyanat-Ester-Harz, Epoxydharze und Acrylatharze.

Die noch nicht vernetzten, jedoch mit einem Vernetzer versehenen elastomeren Bestandteile und die Gewebe-Einlagen werden direkt bei der Herstellung der faserverstärkten Kunststoffteile an den entsprechenden Orten mit in die Form eingelegt. Im Verbundprodukt können als Einlage bevorzugt eingebracht sein: Glasfasern, Nylon, Polyester, Kohlefasern, Viskose, Aramidfasern und/oder Metallfasern. Die Einlage kann in Form eines Gewebes, eines Geleges oder als Pulpe vorgesehen sein.

Bei einer Verwendung eines thermoplastischen Elastomers (TPE) für die Elastomerschicht in Verbindung mit einer duroplastischen Kunststoff-Trägerschicht wird das TPE optional vor dem Zusammenbringen mit dem Duroplast auf eine Temperatur in der Nähe des Erweichungspunkts des TPE erwärmt. Dadurch wird das TPE gegenüber dem Duroplast besser drapierbar. Es lässt sich besser an dessen Kontur und/oder an die Kontur einer Form oder eines Werkzeugs anpassen, das für die Herstellung des Kunststoff-Verbundbauteils verwendet wird.

Durch das Einbringen von weichen elastomeren Schichten kann durch die Auswahl der geeigneten Materialien sowohl eine Schwingungsabsorbtion als auch eine Schwingungsisolation erreicht werden. Die Rissausbreitung wird unterbunden, wobei die Rissausbreitung insbesondere durch in ein Elastomermaterial vollständig eingebettetes Gewebe, Gewirke oder eine Faserstruktur aus Hochleistungsfasern (wie Aramid oder Vectran®, eingetragene Marke der Kuraray Co., Ltd., JP) verhindert wird.

Durch das optionale Einbringen von Flamm-hemmend ausgerüsteten Elastomerschichten wird die Brandausbreitung behindert.

Die Herstellung einer elektrisch ableitenden bzw. Strahlungs-Abschirmenden Variante ist ohne signifikant höhere Materialkosten durch Einbetten leitender Fäden, Streifen oder Gewebe möglich.

Eine besonders vorteilhafte Eigenschaft eines erfindungsgemäßen Kunststoff-Verbundbauteils besteht darin, dass das Kunststoff-Verbundbauteil Energie absorbiert und somit in allen Bereichen einsetzbar ist, in denen Bauteile vor auftreffenden mechanischen Energien und Impulsen geschützt werden müssen oder umgekehrt, in denen Personen oder Gegenstände vor aufprallenden Verbundbauteilen geschützt werden müssen.

Eine weitere vorteilhafte Eigenschaft eines erfindungsgemäßen Verbundbauteils besteht darin, dass das Verbundbauteil ein verbessertes Crash-Verhalten aufweist mit der Folge eines wirkungsvollen Splitterschutzes zur Vermeidung von Verletzungen und einer Vermeidung eines plötzlich auftretenden Totalversagens des Verbundbauteils.

In der bereits vorstehend erwähnten, gattungsgemäßen WO 2006/122749 A1 sind bereits eine ganze Reihe von vorteilhaften Anwendungszwecken für ein erfindungsgemäßes Kunststoff-Verbundbauteil genannt, ohne allerdings im Einzelnen den für den jeweiligen Anwendungszweck besonders vorteilhaften Schichten-Aufbau und den bevorzugten Applikationsbereich detailliert anzugeben:
- Rotorblatt, beispielsweise eines Windrades oder Helikopters
- Tragfläche von Luftfahrzeugen
- Blattfeder, beispielsweise im Fahrzeugbau
- Schlag- oder Geschoss-Hemmendes Bauteil in Schutzkleidungen, wie Protektoren in Motorradbekleidung oder kugelsicheren Westen oder gepanzerten Fahrzeugen
- stabiler Schwingungs-Dämpfender Kern von Sportgeräten, wie Skiern, Snowboards, Schlitten, Bobs, Surfbrettern, Booten, Tennis- oder Eishockeyschlägern
- Lager für Maschinen, Maschinenteile, Brücken und andere Bauwerke und Bauwerksteile
- Gehäuse für hochwertige Konsumgüter, wie Computer oder Notebooks
- Wandung von Rohren, Elastomer-Beschichtete Zylinder oder Rohre
- Verkleidungs- oder Karosserieteil von Fahrzeugen, als Zylinderkopfhaube von Motoren oder als Stoßstange
- Bauteile mit reduzierter Bruch- oder Splitterneigung bei Deformation
- Flammhemmende Verbundteile durch Einbau unbrennbarer elastischer Schichten (z.B. Metall- oder Metallhydroxidpartikel oder halogenierte Paraffine als Zuschlagstoffe im Elastomer)
- Selbstlöschende und Brand-hemmend ausgestattete Gummimischungen

Die gattungsbildende WO 2006/122749 A1 erwähnt weiterhin, dass für bestimmte Anwendungszwecke ein mehrschichtiger Aufbau vorteilhaft ist, wobei vorteilhaft jeweils eine Kunstharzschicht und eine mit einem Vernetzer versehene Elastomerschicht einander abwechseln und wobei vorteilhaft eine oder beide Außenschichten durch Kunstharzschichten gebildet werden. Der Sandwich-Aufbau kann jedoch auch umgekehrt werden, wenn weiche Außenschichten erwünscht sind, wie beispielsweise bei einem Tischtennisschläger, einem Innenverkleidungsteil eines Fahrzeugs oder einem Mouse-Pad.

Die in der gattungsbildenden WO 2006/122749 A1 angegebenen Vorteile gelten prinzipiell auch für die in der vorliegenden Anmeldung beschriebenen Kunststoff-Verbundbauteile:
- Die separate Herstellung eines Harz-Basierenden Festigkeitsträgers ist nicht mehr notwendig.
- Der Aufbau des gesamten Formteils geschieht in einem durchgehenden Arbeitsgang an demselben Arbeitsplatz.
- Eine Verwendung von Haftvermittlern ist nicht nötig.
- Geringerer Zeitaufwand für die Herstellung, dadurch erhebliche Kostensenkung.

Ebenso ist es prinzipiell für die in der vorliegenden Anmeldung beschriebenen Kunststoff-Verbundbauteile vorteilhaft, wenn - wie in der gattungsbildenden WO 2006/122749 A1 beschrieben - die Elastomerschichten wenigstens 0,5 pph ("parts per Hundred") wenigstens eines Vernetzers aus der Gruppe der Peroxide, der Amine und/oder der Bisphenole enthalten und beide, die Trägerschicht und die Elastomerschicht, durch Wärmeeinwirkung oder eine andere Form eines Energieeintrags in einem Arbeitsgang - ohne die Notwendigkeit eines Haftvermittlers - miteinander verbindbar sind. Bei Verwendung eines thermoplastischen Elastomers (TPE), wie Styrol/Ethenbuten/Styrol-Block-Copolymerisat (SEBS) oder Styrol/Butadien/Styrol-Block-Copolymerisat (SBS) oder eines thermoplastischen Elastomers auf der Basis von Polyurethan (TPU) oder eines Polyethylens niedriger Dichte (LDPE) oder Styrol/Butadien-Kautschuk (SBR) mit einem Styrolanteil von mehr als 50 % sind derartige Vernetzer nicht notwendig.

Die vorliegende Erfindung erweitert die bereits vorgeschlagenen Anwendungszwecke dadurch, dass wenigstens eine dünne harte Kunststoff-Außenschicht aus Kunstharz und eine daran anschließende Elastomerschicht gemeinsam mit einer Metall- und/oder Kunststoff-Trägerschicht zu einem Kunststoff-Verbundbauteil verbunden werden, wobei die Kunststoff-Trägerschicht aus einem Faserverstärkten Kunststoff (FVK), einem Carbonfaserverstärkten Kunststoff (CFK) oder einem Glasfaserverstärkten Kunststoff (GFK) gebildet wird. Ein derartiger abwechselnder Schichtaufbau "Hart-weich-hart" hat sich als besonders vorteilhaft für die in den unabhängigen Schutzansprüchen angegebenen Kunststoff-Verbundbauteile herausgestellt, wobei die Schwerpunkte drei grundsätzlich verschiedenen Anwendungszwecken dienen:
- einem Aufprallschutz (Impact-Protection),
- im Rahmen der vorliegenden Teilanmeldung insbesondere einem verbesserten Crash-Verhalten (Splitterschutz, Vermeidung eines Totalversagens eines Bauteils) gemäß Anspruch 1 und weiterhin
- einem verbesserten Verhalten gegenüber Vibrationen.

Von diesen drei Anwendungszwecken können sich bei manchen Bauteilen zumindest zwei überlagern. So ist beispielsweise bei einer Stoßstange oder einer Motorhaube eines Fahrzeugs nicht nur die aktive und passive Sicherheit der Insassen und möglicher mit dem Fahrzeug kollidierender Personen wichtig, sondern auch eine splitterfreie Verformung zum Absorbieren der Aufprall-Energie und eine Reduzierung des Impulses beim Aufprall kleiner Körper (Steinschlag). Motorhauben sollen aber auch keine dröhnenden Geräusche infolge von Schwingungen des Fahrzeugs oder dessen Antriebs erzeugen, so dass bei diesen neben den beiden ersten Aspekten auch der dritte Aspekt der Vibrations-Dämpfung von Bedeutung ist.

In einer für alle Anwendungszwecke vorteilhaften Ausführungsform ist die Kunststoff-Außenschicht aus einem bereits fertig mit Kunstharz getränkten Gewebematerial (Prepreg) gebildet. Alternativ dazu erfolgt die Bildung bzw. Herstellung der Kunststoff-Außenschicht mittels des Harzinfusionsverfahrens.

Alternativ dazu können trockene Fasern (Gewebe, Gelege oder Pulpe) mit in eine Elastomerschicht eingelegt werden, welche sich dann mit der Elastomerschicht und gegebenenfalls auch mit benachbarten Schichten verbinden. Eine in die Elastomerschicht bzw. TPE-Schicht eingebettete Trockenfaserschicht hat dabei eine Wirkung ähnlich einer Folie in einer Verbundglasscheibe: Bei einem Brechen des Verbundbauteils hält sie dessen einzelne Teile zusammen.

Die Kunststoff-Außenschicht ist bevorzugt aus einem faserverstärkten Verbundkunststoff (FVK, CFK, GFK), aus Polyethylen (PE), insbesondere aus einem Polyethylen mit hohem Molekulargewicht (HMW-PE - High Molecular Weight Polyethylene oder UHMW-PE - Ultra High Molecular Weight Polyethylene) oder aus Polytetrafluorethylen (PTFE) gebildet. Die Oberfläche der Kunststoff-Außenschicht ist somit relativ hart und bevorzugt auch sehr glatt. Die Kunststoff-Außenschicht und/oder die Kunststoff-Trägerschicht können alternativ auch von einem so genannten "Organoblech" gebildet werden, einem thermoplastischen Kunststoff mit einer eingebetteten Langfaserverstärkung oder Endlos-Faserverstärkung. Von Langfasern wird in diesem Zusammenhang bei einer Faserlänge von 2 mm bis 50 mm gesprochen; bei einer Faserlänge von über 50 mm spricht der Fachmann von Endlosfasern (siehe DE 10 2007 036 660 A1). Das Kunststoffverbundbauteil lässt sich insbesondere bei Verwendung eines Organoblechs als Kunststoff-Außenschicht und/oder als Kunststoff-Trägerschicht gemeinsam mit seinen anderen Schichten in einem Arbeitsgang durch Pressen oder Tiefziehen herstellen.

In die Elastomerschicht ist bevorzugt wenigstens ein Gewebe oder ein Gewirk oder eine Faserstruktur derart eingebettet ist, dass dessen Fasern zumindest in den besonders belasteten Teilbereichen vollständig vom Elastomer umgeben sind. Die Einbettung erfolgt innerhalb der Elastomerschicht so, dass das Gewebe oder das Gewirk oder die Faserstruktur näher zur Seite einer Zug-Beanspruchung oder Biege-Zugbeanspruchung des Kunststoff-Verbundbauteils angeordnet ist. Das Gewebematerial der Trägerschicht besteht bevorzugt aus Glasfasern, Nylon, Polyester, Kohlefasern, Viskose, Aramidfasern oder Metallfasern. Die Fasern können in Form eines Gewebes, eines Geleges oder einer Pulpe angeordnet sein. Als Kunstharz verwendbar ist besonders bevorzugt Polyesterharz, Phenol-Formaldehyd-Harz, Cyanat-Ester-Harz, Epoxidharz oder Acrylatharz. Das Gewebe oder das Gewirk oder die Faserstruktur bestehen insbesondere bei solchen Kunststoff-Verbundbauteilen, bei denen ein Splittern und ein plötzlich auftretendes Bauteil-Totalversagen unbedingt zu vermeiden ist, besonders bevorzugt aus einer Hochleistungsfaser wie Aramid oder Vectran® (eingetragene Marke der Kuraray Co., Ltd., JP).

Die Elastomerschicht enthält - sofern sie nicht aus TPE besteht - ein Vernetzungssystem, welches je nach verwendetem Elastomer wenigstens einen Vernetzer aus der Gruppe der Peroxide, der Amine und/oder der Bisphenole enthält und eine Reaktion mit dem Kunstharz der Trägerschicht ermöglicht. Alternativ zu einer Wärmebehandlung für eine Vernetzung der Elastomerschicht mit dem Kunstharz der Trägerschicht kann auch eine andere vernetzende Behandlung, beispielsweise mit ultravioletter Bestrahlung (UV-Licht) erfolgen. Auf eine erste Elastomerschicht können weitere Elastomerschichten, falls erforderlich mit unterschiedlicher Festigkeit und Härte aufgebracht werden, die so zusammengesetzt sind, dass sie sich mit der jeweils darunter liegenden Elastomerschicht verbinden. Die wenigstens eine Elastomerschicht besteht besonders bevorzugt aus auf Kautschuk basierenden Werkstoffen. Alternativ dazu kann die wenigstens eine Elastomerschicht auch aus einem thermoplastischen Elastomer (TPE) bestehen.

Nachfolgend sind Ausführungsbeispiele der Erfindung anhand der Zeichnung beschrieben. Die Ausführungsbeispiele gemäß Figur 17 bis 20 sind nicht Gegenstand der vorliegenden Erfindung. Es zeigt:
- Fig. 1: eine schematische Darstellung des grundsätzlichen Schichtaufbaus eines Kunststoff-Verbundbauteils,
- Fig. 2: eine Draufsicht auf einen Rotor-Flügel,
- Fig. 3: eine Schnittdarstellung durch den in Drehrichtung vorne liegenden Kantenbereich des Rotorflügels,
- Fig. 4: eine Schnittdarstellung durch einen als Kanten-Schutzteil oder Splitterschutzteil dienendes Kunststoff-Verbundbauteil,
- Fig. 5: eine Draufsicht auf einen Fahrradlenker,
- Fig. 6: eine Schnittdarstellung durch den mittleren Bereich des Fahrradlenkers gemäß Figur 5,
- Fig. 7: eine Draufsicht auf ein flächiges Kunststoff-Verbundbauteil mit streifenförmigen Schwingungsdämpfungs-Bereichen,
- Fig. 8: einen Schnitt durch einen streifenförmigen Schwingungsdämpfungs-Bereich gemäß Figur 7 in einer ersten Variante,
- Fig. 9: einen Schnitt durch einen streifenförmigen Schwingungsdämpfungs-Bereich gemäß Figur 7 in einer zweiten Variante,
- Fig. 10: einen Schnitt durch ein Kunststoff-Verbundbauteil mit einer flächigen Schwingungsdämpfung in einer ersten Variante,
- Fig. 11: einen Schnitt durch ein Kunststoff-Verbundbauteil mit einer flächigen Schwingungsdämpfung in einer zweiten Variante,
- Fig. 12: einen Schnitt durch ein Kunststoff-Verbundbauteil mit einer flächigen Schwingungsdämpfung in einer dritten Variante,
- Fig. 13: einen Schnitt durch ein Kunststoff-Verbundbauteil mit einer flächigen Schwingungsdämpfung in einer vierten Variante,
- Fig. 14: einen Schnitt durch ein Kunststoff-Verbundbauteil mit einer flächigen Schwingungsdämpfung in Ruheposition,
- Fig. 15: einen Schnitt durch ein Kunststoff-Verbundbauteil mit einer flächigen Schwingungsdämpfung in einer durch Schwingungen ausgelenkten Position,
- Fig. 16: einen Schnitt durch ein schwingungsdämpfendes Kunststoff-Verbundbauteil,
- Fig. 17: einen Schnitt durch ein bi-elastisches Kunststoff-Verbundbauteil im unbelasteten Zustand,
- Fig. 18: das Kunststoff-Verbundbauteil gemäß Fig. 17 bei Belastung von der harten Schichtseite,
- Fig. 19: das Kunststoff-Verbundbauteil gemäß Fig. 17 bei Belastung von der weichen Schichtseite,
- Fig. 20: ein Kunststoff-Verbundbauteil mit einem scharnierartig bewegbaren Verbindungsbereich (Gelenkbereich), und
- Fig. 21: ein Kunststoff-Verbundbauteil mit einer Kernschicht aus einem Elastomer.

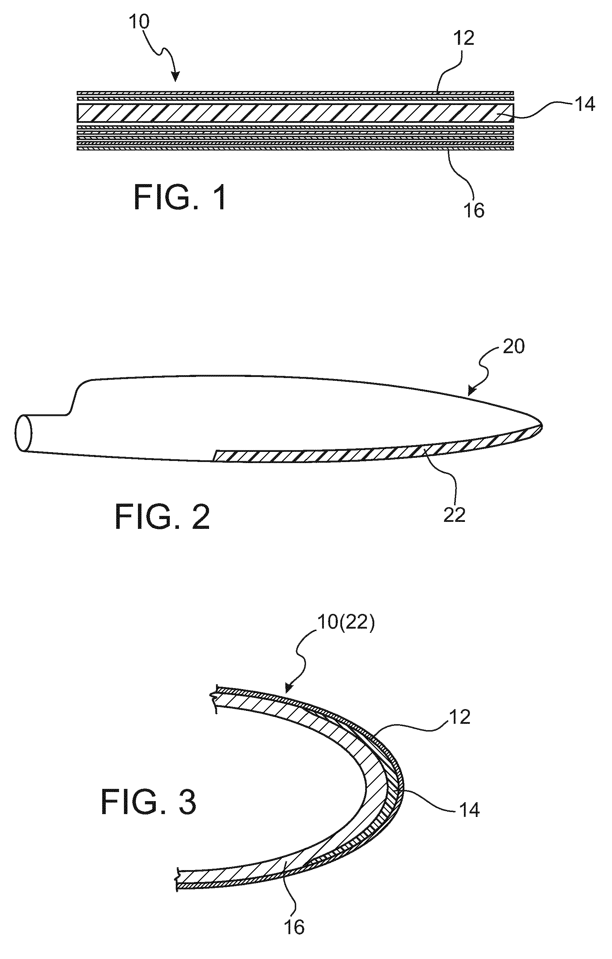

Das in Figur 1 dargestellte Kunststoff-Verbundbauteil 10 besteht aus einer Kunststoff-Außenschicht 12, einer nach innen daran anschließenden Elastomerschicht 14 und einer nach innen an diese anschließenden Kunststoff-Trägerschicht 16.

Die Kunststoff-Außenschicht 12 besteht aus ein oder zwei Faserlagen, die mit flüssigem Kunstharz getränkt sind. Die mit Kunstharz getränkten Faserlagen der Kunststoff-Außenschicht 12 können als vorgefertigtes Bauteil in Form einer mit Kunstharz getränkten Fasermatte (Prepreg) ausgebildet sein oder nach dem Harzinfusionsverfahren hergestellt sein. Die Kunststoff-Außenschicht 12 ist vorzugsweise aus einem faserverstärkten Verbundkunststoff (FVK, CFK, GFK) oder aus Polyethylen (PE), insbesondere aus einem Polyethylen mit hoher Dichte (HMW-PE - High Molecular Weight Polyethylene oder UHMW-PE - Ultra High Molecular Weight Polyethylene) gebildet.

Die Elastomerschicht 14 besteht aus einem der folgenden Stoffe:
aus Ethylen-Propylen- Kautschuk (EPM), Ethylen-Propylen-Dien-Kautschuk (EPDM), Ethylen-Acrylat-Kautschuk (EAM), Fluorkarbon-Kautschuk (FKM), Acrylat-Kautschuk (ACM), Acrylnitril-Butadien-Kautschuk (NBR), optional im Verschnitt mit Polyvinylchlorid (PVC), Hydriertem Nitril-Kautschuk (HNBR), Carboxylat-Nitril-Kautschuk (XNBR), Hydrierter Carboxylat-Nitril-Kautschuk (XHNBR), Naturkautschuk (NR), Ethyl-Vinyl-Acetat (EVA), Chlorsulfonyl-Polyäthylen-Kautschuk (CSM), Chloriertes Polyethylen (CM), Butyl- oder Halobutyl-Kautschuk, Silikon-Kautschuk (VMQ, MVQ), Fluor-Silikon-Kautschuk (FVMQ, MFQ), Chlorhydrin-Kautschuk (CO), Epichlorhydrin-Kautschuk (ECO), Polychlorpren-Kautschuk (CR), einkomponentigem Polyurethan (PU) oder einer Kombination oder einem Verschnitt der vorstehend genannten Stoffe. Alternativ dazu kann die wenigstens eine Elastomerschicht auch aus einem thermoplastischen Elastomer (TPE) bestehen.

Die Elastomerschicht 14 enthält, sofern sie nicht aus einem thermoplastischen Elastomer (TPE) besteht, ein Vernetzungssystem, welches eine Reaktion mit dem Kunstharz der Außenschicht 12 und der Kunststoff-Trägerschicht 16 ermöglicht. Als Vernetzer sind je nach verwendetem Elastomer für die Elastomerschicht 14 folgende Materialien aus wenigstens einer der Gruppen der Peroxide, der Amine und/oder der Bisphenole geeignet:

| **Elastomer** | **Peroxid (Ja/Nein bzw. Coagens**) | **Amin** | **Bisphenol** |
|---|---|---|---|
| Ethylen-Propylen-Kautschuk (EPM); Ethylen-Propylen-Dien-Kautschuk (EPDM) | ja; Methacrylat Acrylat Phenolharz Hexamethylentetramin (HMTA) Hexamethoxymethylmelamin (HMMM) | nein | nein |
| Ethylen-Acrylat-Kautschuk (EAM) | ja | ja | nein |
| Fluorkarbon-Kautschuk (FKM) | ja | ja | ja |
| Acrylat-Kautschuk (ACM) | ja | ja | nein |
| Acrylnitril-Butadien-Kautschuk (NBR), optional im Verschnitt mit Polyvinylchlorid (PVC); Hydrierter Nitril-Kautschuk (HNBR); Hydrierter Carboxylat-Nitril-Kautschuk (XHNBR) | ja; Methacrylat Acrylat Phenolharz Hexamethylentetramin (HMTA) Hexamethoxymethylmelamin (HMMM) | nein | nein |
| Carboxylat-Nitril-Kautschuk (X-NBR) | ja; Peroxid Zinkperoxid | nein | nein |
| Naturkautschuk (NR) | ja; Methacrylat Acrylat Phenolharz Hexamethylentetramin (HMTA) Hexamethoxymethylmelamin (HMMM) | nein | nein |
| Ethyl-Vinyl-Acetat (EVA); Chlorsulfonyl-Polyäthylen-Kautschuk (CSM); chloriertes Polyethylen (CM), z.B. Tyrin® | ja; Methacrylat Acrylat Phenolharz Hexamethylentetramin (HMTA) Hexamethoxymethylmelamin (HMMM) | ja | nein |
| Butyl-Kautschuk (BIIR); Halobutyl-Kautschuk | Bismalein -imide m-Phenylen-bismaleinimid (HVA-2) | nein | nein |
| Silikon-Kautschuk (VMQ, MVQ) | ja (Acrylate) | nein | nein |
| Fluor-Silikon-Kautschuk (MFQ, FVMQ) | ja (Acrylate) | nein | nein |
| Polyurethan (PU, einkomponentig) | ja (Acrylate) | nein | nein |
| Chlorhydrin-Kautschuk (CO) Epichlorhydrin-Kautschuk (ECO) | ja | Thioharnstoff und -derivate u.a. | nein |
| Polychloropren-Kautschuk (CR) | ja | Thioharnstoff und -derivate u.a. | nein |

Der Anteil des Vernetzers bzw. der Vernetzer am Elastomermaterial beträgt etwa zwischen 0,5 bis 15 pph rubber (Teile je 100 Teile Kautschukanteil der Gummimischung), kann aber auch deutlich höher liegen.

Die Kunststoff-Trägerschicht 16 ist bevorzugt aus wenigstens einer Schicht eines Faserverstärkten Kunststoffs (FVK), eines Carbonfaserverstärkten Kunststoffs (CFK) oder eines Glasfaserverstärkten Kunststoffs (GFK) gebildet. Alternativ oder ergänzend kann wenigstens eine Schicht der Kunststoff-Trägerschicht 16 auch aus einem anderen Werkstoff, insbesondere aus Metall, bestehen. Als weitere Alternative kann die Kunststoff-Außenschicht und/oder die Kunststoff-Trägerschicht auch von einem so genannten "Organoblech" gebildet werden, einem thermoplastischen Kunststoff mit einer eingebetteten Langfaserverstärkung oder Endlos-Faserverstärkung.

Bei dem exemplarisch in den Fign. 2 und 3 dargestellten Ausführungsbeispiel dient das Kunststoff-Verbundbauteil zum Schutz gegen aufprallende Gegenstände ("Impact Protection"). Als Beispiel ist in Figur 2 ein Rotorblatt 20 eines Windrades dargestellt, bei dem zumindest im Bereich der in Drehrichtung vorne liegenden Kante ein Kunststoff-Verbundbauteil 22 als Schutz gegen eine witterungsbedingte Beschädigung durch Regentropfen, Staub, Sand, Hagelkörner oder durch Vogelschwärme angeordnet ist. Bei modernen Offshore-Windkraftanlagen betragen die Durchmesser der Windräder derzeit bis zu etwa 126 m, wobei die Umlaufgeschwindigkeit an den Enden der Rotorblätter bis zu 500 km/h erreicht. Falls bei diesen Geschwindigkeiten Regen oder Hagel auf den Rotor einwirkt, können die Rotorblätter durch den Aufschlag ernsthaft beschädigt werden oder durch vorzeitigen Verschleiß die aerodynamischen Eigenschaften des Rotors und damit die Leistung der Windkraftanlage beeinträchtigt werden. Durch das Kunststoff-Verbundbauteil 10 mit seiner unter der dünnen, harten, bevorzugt aus UHMW-PE bestehenden Kunststoff-Außenschicht 12 angeordneten Elastomerschicht 14 wird der auf das eigentliche Rotorblatt 20 einwirkende Aufprall derartiger Gegenstände deutlich gemindert, so dass Beschädigungen wirkungsvoll verhindert werden. Das Rotorblatt 20 kann auch in den übrigen Bereichen durch ein derartiges Kunststoff-Verbundbauteil 10 geschützt sein oder kann sogar vollständig von einem solchen Kunststoff-Verbundbauteil gebildet werden. Alternativ ist auch eine Ausbildung des Kunststoff-Verbundbauteils 10 als Kappe möglich, so wie dies beispielsweise in der DE 10 2008 006 427 A1 anhand eines Erosionsschildes aus Metall beschrieben ist. Die Kappe bedeckt dabei bevorzugt das vordere äußere Viertel bis Fünftel der Länge eines Rotorblatts 20. Dies ist der Bereich, in dem aufgrund der hohen Rotationsgeschwindigkeit die größte Gefahr von Erosion oder Beschädigung besteht. Die harte, glatte Oberfläche der Kunststoff-Außenschicht 12 verhindert neben einer Vereisung auch Korrosion und Ablagerungen am Rotorblatt 20 und reduziert dadurch den Wartungsaufwand auf ein Minimum. Die etwas höheren Kosten bei der Herstellung des Rotorblatts 20 werden durch niedrigere Wartungskosten und verminderte Ausfallzeiten mehrfach aufgewogen. Durch die Erfindung rückt das Ziel eines über eine Laufdauer von 20 Jahren wartungsfreien Betriebs einer Windkraftanlage in greifbare Nähe.

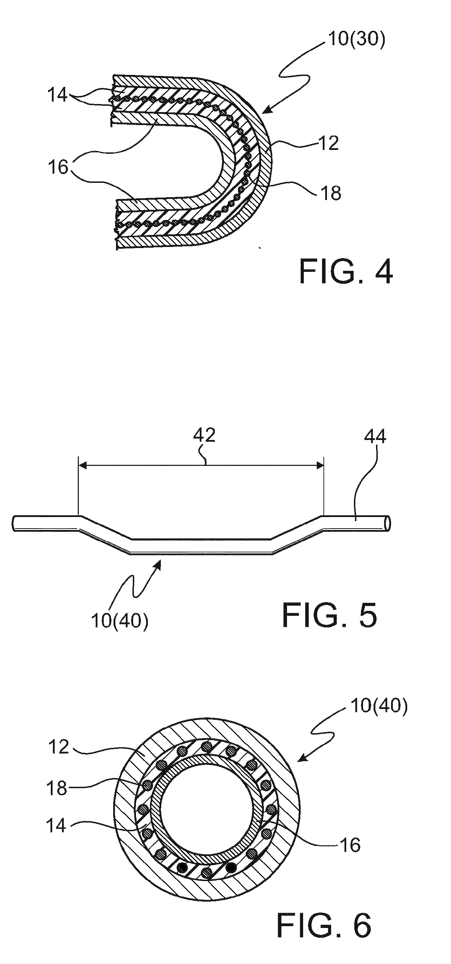

In Figur 4 ist ein Kunststoff-Verbundbauteil 10 als Kantenschutz-Verbundbauteil oder Splitterschutz-Verbundbauteil ausgebildet. An eine Kunststoff-Außenschicht 12 schließt sich nach innen eine Elastomerschicht 14 und eine Kunststoff-Trägerschicht 16 an. In die Elastomerschicht 14 ist ein Gewebe, ein Gewirk oder eine Faserstruktur 18 aus einer Hochleistungsfaser wie Aramid oder Vectran® (eingetragene Marke der Kuraray Co., Ltd., JP) derart eingebettet, dass die Fasern zumindest in den potenziell von einem Stoß beaufschlagten Bereichen vollständig vom Material der Elastomerschicht 14 umgeben sind. Die Einbettung erfolgt bevorzugt so, dass die Faserstruktur 18 innerhalb der Elastomerschicht 14 näher an der durch Zug- oder Biege-Zug beanspruchten Seite des Kunststoff-Verbundbauteils 10 - im vorliegenden Fall also näher an der Kunststoff-Außenschicht 12 - angeordnet ist. Durch die Einbettung in das Elastomermaterial wird die Bruch- und Reißfestigkeit der Faserstruktur 18 deutlich erhöht. Versuche haben gezeigt, dass ein derartiges Kunststoff-Verbundbauteil 10 in der Lage ist, eine Aufprallenergie ohne Beschädigung des Bauteils aufzunehmen, die über 400% des sonst üblichen Wertes beträgt. Im Crash-Fall wird die Gefahr eines Splitterns oder eines Weiterreißens eines gebrochenen Bauteils drastisch reduziert.

Das in Figur 1 dargestellte flächige Kunststoff-Verbundbauteil 10 und das in Figur 4 dargestellte Kantenschutz-Verbundbauteil bzw. Splitterschutz-Verbundbauteil 30 decken eine große Zahl möglicher Anwendungsfälle für Bauteile ab, die einem Aufprall eines Körpers ausgesetzt sind. Dies sind erfindungsgemäß beispielsweise:
- die Vorderkante eines Flügels, einer Tragfläche oder eines Leitwerks eines Luftfahrzeugs, oder
- die in Drehrichtung liegenden Vorderkante eines Rotorblattes eines Hubschraubers oder eines Windrades, oder
- ein Karosseriebauteil eines Fahrzeugs, wie eine Stoßstange oder eine Motorhaube, oder
- ein aufwirbelnden Gegenständen ausgesetztes Bauteil eines Fahrzeugs, wie einem Unterbodenschutzteil eines Landfahrzeugs oder Schienenfahrzeugs, einer Fahrwerksstrebe, einem Lenkgestänge, einer Antriebswelle oder Kardanwelle, einem Tretlager oder einer Kettenstrebe eines insbesondere mit einem Carbon-Rahmen versehenen Mountain-Bikes, oder
- eine Innenverkleidung eines Land-, Wasser-, Luft- oder Raum-Fahrzeugs, oder
- die Innenseite eines zu Wartungszwecken begehbaren Hohlraums von Land-, Wasser-, Luft- oder Raum-Fahrzeugen zum Schutz vor Beschädigungen durch herunterfallende Werkzeuge, oder
- der Ladung zugewandte Oberflächen von offenen oder geschlossenen Transporträumen, wie Laderäumen von Transportern und Lastkraftwagen oder Containern, oder
- der vordere Rumpfbereich oder der Kielbereich eines Wasserfahrzeugs, wie eines Sportbootes, eines Speed-Bootes oder eines Kajaks, oder
- stark beanspruchte Nutzflächen von Sportgeräten, wie der Schaufelbereich von Eishockey-Schlägern, die Boden-Kontaktflächen von Nordic-Walking-Stöcken oder Ski-Stöcken oder Paddelblätter von Kanu- oder Kajakpaddeln, oder
- ein Verstärkungsteil eines gepanzerten Fahrzeugs
wobei die vorstehende Aufzählung nur exemplarisch und keinesfalls erschöpfend zu verstehen ist.

Das in Figur 4 dargestellte Kunststoff-Verbundbauteil bzw. Splitterschutz-Verbundbauteil 10 weist durch die in die Elastomerschicht 14 eingebettete Faserstruktur 18 auch ein deutlich verbessertes Crash-Verhalten auf, da bei einem Bruch weniger scharfkantige Bruchkanten und weniger lose Bruchstücke entstehen und die Fasern eine Reststabilität gewährleisten. Erfindungsgemäße Anwendungsfälle hierfür sind beispielsweise:
- ein Innen-Verkleidungsteil eines Fahrzeugs, wie eine Tür-Verkleidung, oder
- ein äußeres Karosseriebauteil, wie ein Spoiler, ein Kotflügel, ein Fahrzeugdach, eine Heckklappe, eine Motorhaube, eine Crash-Nase oder ein Seitenteil eines Rennwagens, oder
- ein Rahmen, ein Lenker, ein Lenker-Vorbau oder eine Sattelstütze eines Zweirads, ein Tennisschläger, eine Schiene oder eine Fersenkappe von Inline-Skates, ein Body-Protektor oder ein Schutzhelm für berufliche oder freizeitmäßige Schutzkleidung (Feuerwehr, Polizei, Militär und Sicherheitsdienste; Fahrrad-, Motorrad-, Skater- oder Skihelme oder Protektoren)
wobei die vorstehende Aufzählung nur exemplarisch und keinesfalls erschöpfend zu verstehen ist.

In den Fign. 5 und 6 ist ein weiterer Anwendungsfall für ein erfindungsgemäßes Kunststoff-Verbundbauteil bzw. Splitterschutz-Verbundbauteil 10 exemplarisch anhand eines Fahrradlenkers 40 dargestellt. Bei dieser Anwendungsgruppe handelt es sich um Gegenstände, bei denen in jedem Falle ein Totalversagen des Bauteils ausgeschlossen werden soll. Zu diesem Zweck besteht der Fahrradlenker 40 aus einer Kunststoff-Außenschicht 12, die bevorzugt von einer Carbonfaserstruktur (CFK) gebildet wird. An diese schließt sich zumindest im inneren Verbundbauteilbereich 42 des Fahrradlenkers 40 nach innen eine Elastomerschicht 14 an, in die ein Gewebe, ein Gewirk oder eine Faserstruktur 18, bevorzugt aus einer Hochleistungsfaser wie Aramid oder Vectran® (eingetragene Marke der Kuraray Co., Ltd., JP) derart eingebettet ist, dass die Fasern vollständig vom Material der Elastomerschicht 14 umgeben sind. An die Elastomerschicht 10 schließt sich nach innen optional eine weitere dünne Kunststoff-Trägerschicht 16 an, die ebenfalls bevorzugt von einer Carbonfaserstruktur (CFK) gebildet ist. Sollte bei einer extremen Belastung (z.B. bei einem Downhill-Racing mit einem Mountainbike) die Kunststoff- Außenschicht 12 des Fahrradlenkers 40 einreißen oder brechen, so wird der Fahrradlenker 40 dennoch durch die in die Elastomerschicht 14 eingebettete Faserstruktur 18 vor einem plötzlichen vollständigen Versagen geschützt. Der Fahrer kann den sich anbahnenden Bruch seines Lenkers rechtzeitig wahrnehmen und seine Geschwindigkeit entsprechend reduzieren, ohne dabei einen Sturz zu erleiden, da das Fahrrad noch lenkbar bleibt. Erfindungsgemäße Anwendungsgegenstände aus dieser Gruppe sind beispielsweise:
- ein Rotorblatt oder eine Tragfläche eines Sportflugzeugs, oder
- eine Prothese oder Orthese, oder
- ein Rahmen, ein Lenker, ein Lenker-Vorbau oder eine Sattelstütze eines Zweirads, ein Tennisschläger, eine Schiene oder eine Fersenkappe von Inline-Skates, ein Body-Protektor oder ein Schutzhelm (Motorradhelm, Fahrradhelm, Arbeitsschutzhelm, Feuerwehrhelm und dergleichen), oder
- ein vorderer Bereich (Einlassbereich und erste Kompressionsstufe eines Triebwerks oder einer Turbine, die besonders der Gefahr von Vogelschlag oder bei kleineren Maschinen auf schlechten Pisten der Gefahr von Steinschlägen ausgesetzt sind).
wobei die vorstehende Aufzählung nur exemplarisch und keinesfalls erschöpfend zu verstehen ist.

Derartige Kunststoff-Verbundbauteile 10 mit einer in eine Elastomerschicht 14 eingebetteten Faserstruktur 18 sind auch sehr gut als Schutz vor Schuss-, Stich-und Stoßverletzungen im Bereich des Personenschutzes (kugelsichere Weste, Schutzschilde) und bei entsprechenden Sportarten (Fechten, Reiten, Motorradfahren, Motocross, Eishockey) in die Schutzkleidung zu integrieren oder in entsprechenden Protektoren zu verarbeiten.

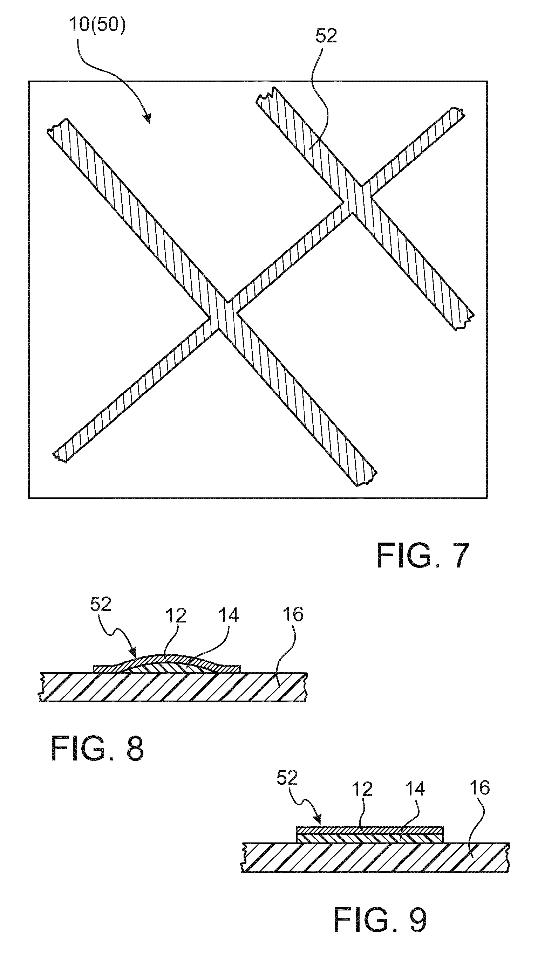

In den Fign. 7 bis 15 ist ein weiterer Komplex von erfinderischen Anwendungsfällen für Kunststoff-Verbundbauteile 10 gezeigt. Es handelt sich dabei um Bauteile, bei denen Vibrationen gedämpft und/oder Resonanzen verhindert oder vermindert werden sollen und/oder eine Reduzierung von akustischen Schwingungen erreicht werden soll. Hierzu ist bei einem flächigen Kunststoff-Verbundbauteil 10, beispielsweise einer Motorhaube 50, zumindest in Teilbereichen, wie den in Figur 7 angedeuteten Streifen, wenigstens ein Dämpfungs-Verbundbauteil 52 vorgesehen. Auf einer Kunststoff-Trägerschicht 16 ist in den Teilbereichen des Dämpfungs-Verbundbauteils 52 eine Elastomerschicht 14 und über dieser eine Kunststoff-Außenschicht 12 angeordnet. Die Anordnung kann auch umgekehrt sein, so dass die in den Fign. 8 und 9 mit 16 bezeichnete Schicht die Kunststoff-Außenschicht 12 bildet, an die sich nach innen die Elastomerschicht 14 und anstelle der Schicht 12 die Kunststoff-Trägerschicht 16 anschließt. Diese mögliche Umkehr zeigt, dass auch die Kunststoff-Außenschicht 12 prinzipiell für alle in dieser Anmeldung beschriebenen Anwendungsgegenstände als Kunststoff-Trägerschicht fungieren kann, während die innenliegende Schlicht 16 in diesem Falle keine tragende Funktion hat, sondern lediglich die Schwingungen dämpfende Elastomerschicht 14 nach innen begrenzt. Dabei ist in Figur 8 die Elastomerschicht 14 vollständig von der Schicht 12 abgedeckt, während in Figur 9 die Elastomerschicht 14 zu den Seiten offen ist.

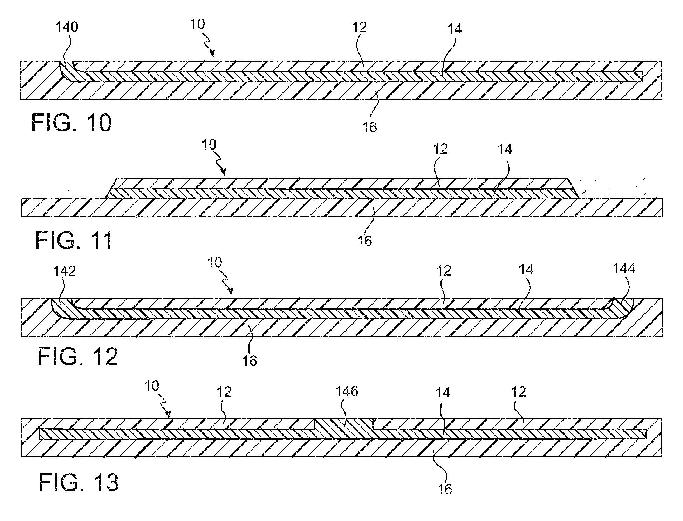

In den Fign. 10 bis 13 sind mehrere Ausführungsbeispiele für flächige Kunststoff-Verbundbauteile 10 dargestellt, die in der Lage sind, Vibrationen, Resonanzen und akustische Schwingungen wirkungsvoll zu dämpfen. In Figur 10 ist eine Elastomerschicht 14 derart zwischen einer Kunststoff-Außenschicht 12 und einer Kunststoff-Trägerschicht 16 eingebettet, dass sie mit einem Austrittsbereich 140 an der Kunststoff-Außenschicht 12 hervortritt.

In Figur 11 ist die Elastomerschicht 14 nach beiden Seiten offen auf die Kunststoff-Trägerschicht 16 aufgesetzt und nach oben durch eine Kunststoff-Außenschicht 12 abgedeckt.

In Figur 12 ist die Elastomerschicht 14 zwischen einer Kunststoff-Außenschicht 12 und einer Kunststoff-Trägerschicht 16 derart eingebettet, dass sie mit zwei Austrittsbereichen 142, 144 mit der Kunststoff-Außenschicht 12 in Verbindung steht.

In Figur 13 ist die Elastomerschicht 14 zwischen einer Kunststoff-Außenschicht 12 und einer Kunststoff-Trägerschicht 16 derart eingebettet, dass sie mit einem zentralen Austrittsbereich 146 mit der Oberfläche der Kunststoff-Außenschicht 12 in Verbindung steht.

Für die Ausführungsbeispiele gemäß Figur 10 bis 13 gilt, was schon weiter oben für die Fign. 7 bis 9 gesagt wurde: die Bezeichnungen Kunststoff-Außenschicht 12 und Kunststoff-Trägerschicht 16 sind auch hier austauschbar, so dass auch eine Schwingungsdämpfung an einem Bauteil möglich ist, dass nach außen vollständig glatt ist. In diesem Falle sind die Austrittsbereiche 140, 142, 144 und 146 an der Innenseite des jeweiligen Kunststoff-Verbundbauteils 10 angeordnet.

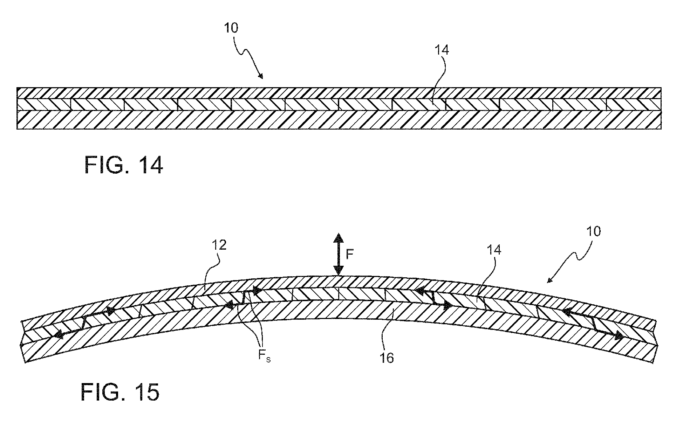

In den Fign. 14 und 15 ist verdeutlicht, auf welche Weise die Energie F von mechanischen oder akustischen Schwingungen im Kunststoff-Verbundbauteil 10 durch senkrecht dazu angeordnete Scherkräfte F_{S} absorbiert wird. Die Elastomerschicht 14 nimmt an den Grenzen zur Kunststoff-Außenschicht 12 und zur Kunststoff-Trägerschicht 16 die Energie F der Schwingungen auf und wandelt diese in senkrecht dazu angeordnete Scherkräfte F_{S} um. Figur 14 zeigt das Kunststoff-Verbundbauteil 10 in seiner Ruheposition, während das Kunststoff-Verbundbauteil 10 in Figur 15 durch die Kraft F einer Schwingung in eine Richtung ausgelenkt wurde.

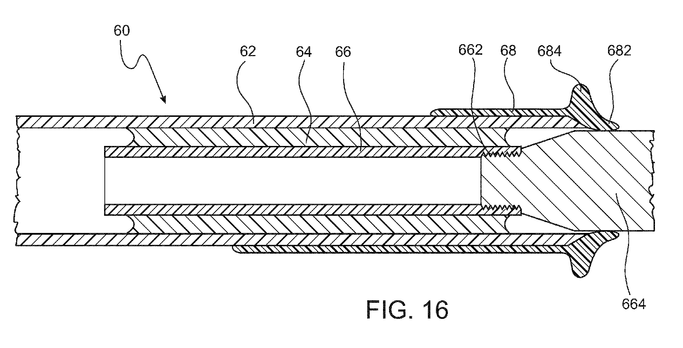

In Fig. 16 ist zumindest ein Griffstück eines Stiels eines pneumatischen Hammers als schwingungsdämpfendes Kunststoff-Verbundbauteil 60 ausgebildet. Der rohrförmige Stiel des Pneumatischen Hammers mit einem Außendurchmesser von etwa 50 mm weist eine Kunststoff-Außenschicht 62 auf, die aus einem der im Patentanspruch 5 genannten Materialien, bevorzugt aus einem faserverstärkten Verbundkunststoff, insbesondere Carbonfaser verstärktem Kunststoff (CFK) gebildet ist. An die Kunststoff-Außenschicht 62 schließt sich bevorzugt im Griffbereich auf eine Länge von etwa 15 cm eine Elastomer- oder Gummischicht 64 an, die aus einem der in den Patentansprüchen 6 bis 11 genannten Materialien oder aus thermoplastischem Elastomer (TPE) gebildet ist. Die Elastomer- oder Gummischicht 64 kann auch aus mehreren derartigen Schichten gebildet sein. Innen schließt sich an die Elastomer- oder Gummischicht 64 eine Kunststoff-Trägerschicht 66 an, deren Material bevorzugt dem der Kunststoff-Außenschicht 62 entspricht. Die rohrförmige Kunststoff-Trägerschicht 66 bildet an einem Ende eine Werkzeugaufnahme 662, die im gezeigten Ausführungsbeispiel als Gewinde ausgebildet ist und ein Werkzeug 664 haltert. Das Gewinde ist im gezeigten Ausführungsbeispiel unmittelbar an der Kunststoff-Trägerschicht 66 ausgebildet. An Stelle dessen kann auch ein metallenes Gewindestück in die Kunststoff-Trägerschicht 66 eingebettet sein. Abweichend vom gezeigten Ausführungsbeispiel kann die Werkzeugaufnahme 662 auch als Bajonettverschluss oder als Konushalterung ausgebildet sein. Optional ist zusätzlich ein Griffbereich 68 aus einem weicheren Elastomermaterial an der Kunststoff-Außenschicht 62 angeordnet. Diese sorgt für ein sicheres Halten des Pneumatik-Hammers durch den Bediener und eine zusätzliche Entkopplung von Schwingungen. Bevorzugt sind alle Schichten 62, 64, 66 und 68 gleichzeitig in einem Arbeitsgang in einer Form zu dem gezeigten Kunststoff-Verbundbauteil verbunden worden.

Am Griffbereich 68 ist bevorzugt eine Dichtlippe 682 angeformt, die das Innere des Stiels mit den darin angeordneten Pneumatik-Komponenten vor Schmutz und Staub schützt. Weiterhin ist an dem dem Werkzeug 664 zugewandten Ende des Griffbereichs 68 bevorzugt ein Handschutz 684 in Form einer nach außen gestülpten Lippe angeformt, die Hände des Benutzers vor Verletzungen schützt.

Wesentlich ist, dass die vom Werkzeug 664 beim Auftreffen auf das zu bearbeitende Material (Steine, Beton, Asphalt, Fiesen) als Reaktion entstehenden Schwingungen durch die Elastomerschicht 64 stark entkoppelt oder gedämpft werden, so dass der den Griffbereich umfassende Bediener weitestgehend von diesen Schwingungen verschont wird.

Derselbe in Fig. 16 dargestellte Aufbau eignet sich ebenso für eine Sattelstütze eines Fahrrads. In diesem Falle ist an der Aufnahme 662 anstelle des Werkzeugs der Sattel befestigt, während der Stiel mit der Kunststoff-Außenschicht 62 zur Befestigung am Rahmenrohr dient. Die Elastomerschicht 64 entkoppelt Schwingungen, die sonst durch Fahrbahnunebenheiten auf den Sattel einwirken würden.

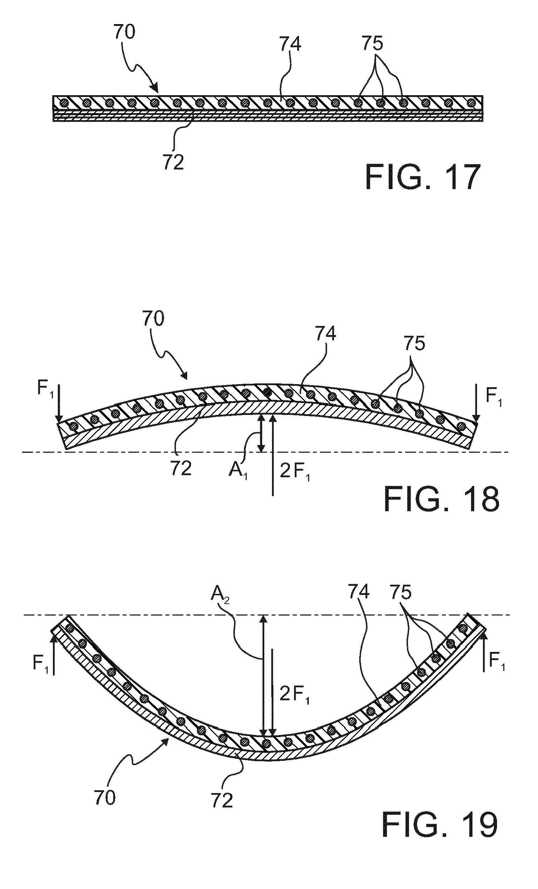

In den Fign. 17 bis 19 ist ein Kunststoff-Verbundbauteil 70 gezeigt, dass aus einer relativ dünnen, flexiblen Kunststoff-Außenschicht 72 und einer damit verbundenen Elastomerschicht 74 mit einer eingebetteten bzw. eingelagerten Gewebe- bzw. Faserschicht 75 gebildet wird. Die Kunststoff-Außenschicht 72 besteht aus einem der im Patentanspruch 5 genannten Materialien. Sie ist bevorzugt als dünne Prepregschicht oder SMC-Schicht ausgebildet. Die Elastomerschicht 74 wird aus einem der in den Patentansprüchen 6 bis 11 genannten Materialien gebildet. Die in das Elastomermaterial der Elastomerschicht 74 eingebettete Gewebe-, Gewirk-oder Faserschicht 75 besteht bevorzugt aus einer Hochleistungsfaser wie Aramid oder Vectran®. Das Kunststoff-Verbundbauteil 70 weist eine Flexibilität in beiden Biegerichtungen auf, wobei diese bei gleicher Kraft zu unterschiedlichen Auslenkungen A₁ bzw. A₂ führt. In Fig. 18 bewirkt eine in Bauteilmitte von der harten Seite der Kunststoff-Außenschicht 72 her angreifende Kraft 2xF1, die in den seitlichen Lagerkräften F₁ ihre Reaktionskraft findet, eine Auslenkung A₁, die deutlich kleiner ist, als eine Auslenkung A₂, die gemäß Fig. 19 durch eine gleich große Kraft 2xF1 entsteht, die von der weicheren Seite von der Elastomerschicht 74 her auf die Mitte des Kunststoff-Verbundbauteils 70 einwirkt. Ein derartiges unterschiedliches Biegeverhalten schafft völlig neue Anwendungsmöglichkeiten, beispielsweise bei Blattfedern, bei aerodynamisch oder hydrodynamisch belasteten Bauteilen, die sich bei einer Druck- bzw. Sogeinwirkung auf unterschiedlichen Seiten "selbsttätig" unterschiedlich stark verformen. Als Anwendung kommen beispielsweise Spoiler an Hochgeschwindigkeitsfahrzeugen, Flügel und Tragflächen von Luftfahrzeugen, Propeller, Turbinen oder ähnliches in Betracht.

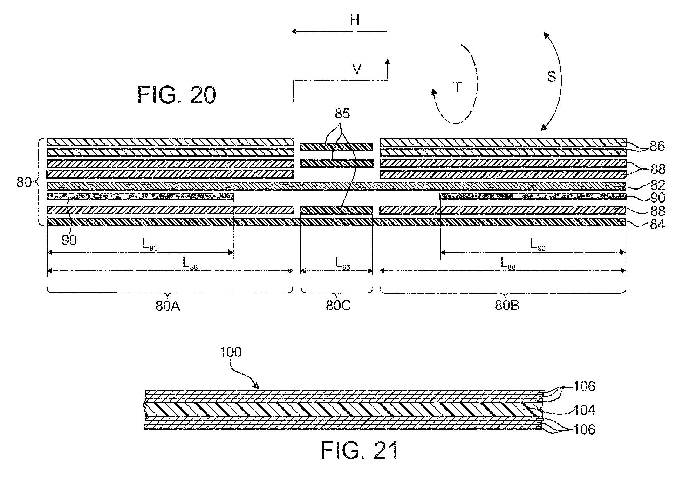

In Fig. 20 ist ein Kunststoff-Verbundbauteil 80 gezeigt, das im mittleren Bereich einen flexiblen Gelenkbereich 80C aufweist. Das dargestellte hochfeste Kunststoff-Verbundbauteil zur Verwendung im Automobilrennsport weist zwei Lagen eines Carbon-Prepreg 86, darunter zwei Lagen eines Glas-Prepreg 88, darunter eine Lage aus einem Glasfasergewebe 82, darunter einen Klebstofffilm 90 und darunter eine Elastomerschicht 84 auf. Die einzelnen Lagen bzw. Schichten sind in der Darstellung in Fig. 20 vertikal voneinander beabstandet, um diese deutlicher unterscheiden zu können. In der Realität bilden alle diese Schichten eng aufeinanderliegend und miteinander verbunden das Kunststoff-Verbundbauteil 80. An Stelle der Glas-Prepreg-Schichten 88 können auch weitere Carbon-Prepreg-Schichten 86 vorgesehen sein. Während das Glasfasergewebe 82 und die Elastomerschicht 84 im gezeigten Ausführungsbeispiel über den Gelenkbereich hinweg durchlaufen, sind die Lagen aus Carbon-Prepreg 86 und Glas-Prepreg 88 im Gelenkbereich 80C unterbrochen und werden hier durch mit ihren Enden verbundene Elastomer-Abschnitte 85 überbrückt. Dadurch entsteht im mittleren Gelenkbereich 80C eine flexible Verbindung der rechts und links davon angeordneten, die Carbon-Prepreg-Schichten 86 und die Glas-Prepreg-Schichten 88 umfassenden starren Bereiche 80A bzw. 80B des Kunststoff-Verbundbauteils 80. Im Ausführungsbeispiel erlaubt das Gelenk folgende drei Freiheitsgrade:
- Eine Schwenk- oder Knickbewegung S des rechten starren Teils 80B relativ zum linken starren Teil 80B,
- einen vertikalen Versatz V des rechten starren Teils 80B relativ zum linken starren Teil 80B, und schließlich
- eine horizontale Verschiebung H des rechten starren Teils 80B hin zum linken starren Teil 80B.

Wird die Schicht aus Glasfasergewebe 82, die je nach Anforderung auch durch ein Carbonfasergewebe, Aramidfasergewebe oder Vectran®-Gewebe (eingetragene Marke der Kuraray Co., Ltd., JP) ersetzt werden kann, im Gelenkbereich 80C unterbrochen oder durch ein Gewebe aus elastischen Fasern ersetzt, ist zusätzlich auch eine begrenzte horizontale Verschiebung des rechten starren Teils 80B weg vom linken starren Teil 80B möglich. Sofern der Gelenkbereich 80C nur durch eine oder mehrere Elastomerschichten 84 bzw. 85 gebildet wird, ist zusätzlich auch eine begrenzte Torsionsbewegung T des rechten starren Teils 80B relativ zum linken starren Teil 80B in die Zeichenebene hinein bzw. aus dieser heraus möglich. Die stärkste Beschränkung der Beweglichkeit des Gelenks 80C ergibt sich, wenn wenigstens eine dünne Kunstharzschicht (Carbon-Prepreg-Schicht 86 oder Glas-Prepreg-Schicht 88) den Gelenkbereich 80C überspannt. Dann ist nur noch eine begrenzte Schwenkbewegung S des rechten starren Teils 80B relativ zum linken starren Teil 80B möglich. In diesem Fall nähert sich der fünfte Anwendungsfall des Kunststoff-Verbundbauteils 80 dem vierten Anwendungsfall des Kunststoff-Verbundbauteils 70 gemäß den Figuren 17 - 19. Auch zur Herstellung eines scharnierartigen Gelenkbereichs an einem Kunststoff-Verbundbauteil 80 kann der dort beschriebene Verbund aus einer sehr dünnen harten Kunstharzschicht 72 und einer mit einer Gewebe- bzw. Faserschicht 75 versehenen Elastomerschicht 74 verwendet werden. Die möglichen Anwendungsbereiche sind sehr vielfältig:
- Tür- oder Klappenscharniere, beispielsweise für Gebäude, Möbel oder Fahrzeuge, aber auch für Koffer, Kisten, Container oder andere Behältnisse, wobei die längs der Scharnierkante durchgehend dichte Gestaltung des Scharnierbereichs in diesem Bereich den Durchtritt von Luft, Flüssigkeit oder Partikeln verhindert
- Dichtungen, die sich dreidimensionalen Konturen anpassen müssen
- Knickbereichen von Platten oder Schläuchen
- Kompensatoren für den Ausgleich eines Höhen- oder Seitenversatzes zweier Bauteilflächen
- aerodynamisch vorteilhaft gestalteter Übergang durch Vermeidung von Spalten zwischen zwei Bauteilen
- Gebäudeverkleidungen in Eckbereichen ("weicher Kantenschutz" z.B. an Pfeilern oder Mauerecken von Tiefgaragen)
- Anlenkung von bewegbaren Klappen, wie Landeklappen von Flugzeugen, Strömungslenkende Klappen an Stauwehren, Rotorblätter von Hubschraubern
- flexible Aufhängungen (z.B. im Motorsport)
wobei die vorstehende Aufzählung nur exemplarisch und keinesfalls erschöpfend zu verstehen ist.

Für stark belastete Bauteile, beispielsweise Karosserieteile von Motorsport-Fahrzeugen, sind Kunststoff-Verbundbauteile 100 vorteilhaft, bei denen gemäß Fig. 21 eine Kernschicht 104 aus einem Elastomer in der Mitte, das heißt im Bereich der neutralen Faser des Kunststoff-Verbundbauteils 100 angeordnet ist. An die Kernschicht 104 schließen sich nach außen bevorzugt mehrere Schichten aus Carbonfaser-Prepreg 106 (CFK) und/oder aus Glasfaser-Prepreg (GFK) und/oder aus anderen Faser-Verbund-Kunststoffschichten (FVK) an, die eine glatte, harte Oberfläche bilden. Im Ausführungsbeispiel sind auf jeder Seite der Kernschicht 104 drei relativ dünne Carbonfaser-Prepreg-Schichten 106 vorgesehen. Die Kernschicht 104 macht das Bauteil gegenüber einem reinen CFK- oder GFK-Bauteil deutlich leichter, da das spezifische Gewicht der relativ dicken Elastomerschicht nur etwa 1 g/ cm³ beträgt, während CFK ein spezifisches Gewicht von etwa 1,8 g/ cm³ und GFK ein spezifisches Gewicht von etwa 2,0 g/ cm³ aufweist.

Verglichen mit anderen bekannten Kunststoff-Verbundbauteilen, bei denen eine extrem leichte Kernschicht mit einem Distanzstege zwischen zwei Decklagen aufweisenden Sandwichaufbau ("Honeycomb-Struktur", beispielweise aus Zellulose oder Pappe) verwendet wird, liegt das Gewicht der erfindungsgemäßen Kernschicht 104 aus einem Elastomer zwar höher; das erfindungsgemäße Kunststoff-Verbundbauteil 100 mit der Kernschicht 104 aus einem Elastomer weist aber diesen extrem leichten Verbundbauteilen gegenüber deutliche Vorteile auf in Bezug auf das Impact-Verhalten und den Vibrationsschutz bzw. das Dämpfungsverhalten gegen Bauteilschwingungen. Durch die Integration von Hochleistungsfasern in die neutrale Elastomerschicht ist ein Splitterschutz zusätzlich integrierbar.

Die Nutz-Oberflächen eines erfindungsgemäßen Kunststoff-Verbundbauteils lassen sich in einfacher Weise an den gewünschten Anwendungszweck anpassen, wobei jeweils im Unterschied zu bekannten Verbundbauteilen eine Verbindung aller Schichten in einem einzigen Arbeitsgang erfolgt. Die Nutz-Oberfläche des Kunststoff-Verbundbauteils kann von der glatten, harten und verkratzfesten Kunststoff-Außenschicht gebildet werden, wo es auf möglichst geringe Friktion und gute Gleiteigenschaften (z.B. bei Skiern oder Snowboards), auf aerodynamische oder hydrodynamische Eigenschaften (z.B. bei Tragflächen oder bei Rümpfen von Luft oder Wasserfahrzeugen), auf Erosions-, Korrosions-, Abrasions- und Witterungsschutz (z.B. bei Flügeln von Helikoptern oder Windrädern, bei Außenpaneelen oder Außenverkleidungsteilen von Gebäuden oder Fahrzeugen), oder auf eine Vermeidung eines Anhaftens von Medien oder Fremdkörpern (z.B. bei Behältern von Rührgeräten, bei Schwimmbecken oder Kläranlagenbassins oder bei Schiffsrüpfen) ankommt.

Umgekehrt kann die Nutz-Oberfläche eines erfindungsgemäßen Kunststoff-Verbundbauteils mit einer friktionserhöhenden weichen Schicht aus einem Elastomer oder TPE versehen sein, wenn dies die Haptik (z.B. Griffstücke, Lenkräder, Schalter und andere Bedienelemente) oder die Rutschfestigkeit (z.B. Oberflächen von Surfboards, Innenauskleidung von Stauräumen, Trittplatten in Ein-und Ausstiegsbereichen von Fahrzeugen) erfordert.

Ein weiteres Anwendungsgebiet für ein erfindungsgemäßes Kunststoff-Verbundbauteil sind Wandungen von fluidführenden Behältern oder Rohrleitungen. Durch die eingebettete Elastomerschicht weisen derartige Behälter oder Rohre einen hervorragenden Berstschutz aus. Insbesondere in Verbindung mit einer flammhemmenden Ausstattung sind derartige Rohre und Behälter beispielsweise zur Aufbewahrung und Führung chemisch aggressiver oder hochexplosiver Flüssigkeiten bestens geeignet. Eine auf der Medium-führenden Seite angeordnete zusätzliche Beschichtung aus einem Gummi oder einem Gummi ähnlichen Elastomer sorgt für die nötige Medien-Resistenz derartiger Behälter oder Leitungen. Mögliche Anwendungsgebiete sind Treibstoff- oder Öltanks in Fahrzeugen aller Art, insbesondere auch in Flugzeugen, Helikoptern oder Schiffen, auch für militärische Einsatzzwecke, Druckluftbehälter oder -leitungen, aber auch Tanks und Leitungen für Wasser, Säfte, sonstige Getränke, Milchprodukte oder andere Lebensmittel, wobei sich hier ein thermoplastisches Elastomer (TPE) als Beschichtung auf der Medium-führenden Seite besonders gut eignet.

Das Kunststoff-Verbundbauteil 10, 22, 42, 50, 60, 70, 80 bzw. 100 mit der Kunststoff-Außenschicht 12, 62, 72, 86 bzw. 106 und der Metall- bzw. Kunststoff-Trägerschicht 16, 66 bzw. 88 sowie der dazwischen angeordneten Elastomerschicht 14, 64, 74; 84, 85 bzw. 104 wird zur Herstellung einer Behandlung durch einen Energie-Eintrag unterzogen. Dieser kann beispielsweise durch eine Wärmebehandlung in einem Ofen, einem Autoklaven, einer beheizten Presse oder einem beheizten Tiefziehwerkzeug, einer Mikrowellen-Anlage, einer Hochleistungs-Lichtstrahleranlage und/oder einem beheizbaren Tisch erfolgen. Dabei liegt die Prozess-Temperatur im Bereich von etwa 80 Grad Celsius bis etwa 200 Grad Celsius, bevorzugt bei etwa 130 Grad Celsius. Die Prozessdauer beträgt dabei etwa 5 Stunden. Die Prozessdauer kann jedoch je nach Kundenwunsch im angegebenen Temperaturbereich von etwa 10 Minuten bis zu etwa 8 Stunden variieren. Alternativ dazu wird das Kunststoff-Verbundbauteil 10 einer anderen vernetzenden Behandlung, beispielsweise mit UV-Licht unterzogen. Dabei vernetzt die wenigstens eine Elastomerschicht 14 mit dem Kunstharz der Kunststoff-Außenschicht 12 und der Kunststoff-Trägerschicht 16. Die Kunststoff-Außenschicht 12, die Kunststoff-Trägerschicht 16 und die Elastomerschicht bzw. Elastomerschichten 12 sind danach unlösbar miteinander verbunden.

### Bezugszeichenliste

- 10: Kunststoff-Verbundbauteil
- 12: Kunststoff-Außenschicht
- 14: Elastomerschicht
- 140: Austrittsbereich
- 142: Austrittsbereich
- 144: Austrittsbereich
- 146: Austrittsbereich
- 16: Kunststoff-Trägerschicht
- 18: Gewebe/ Gewirk/ Faserstruktur
- 20: Rotorblatt
- 22: Kunststoff-Verbundbauteil (an 20)
- 30: Kantenschutz-Verbundbauteil
- 40: Fahrradlenker
- 42: Verbundbauteilbereich (von 40)
- 44: Lenkerende (Griffbereich)
- 50: Motorhaube
- 52: Dämpfungs-Verbundbauteil
- 60: Kunststoff-Verbundbauteil
- 62: Kunststoff-Außenschicht
- 64: Elastomerschicht
- 66: Kunststoff-Trägerschicht
- 662: Werkzeugaufnahme
- 664: Werkzeug
- 68: Griffbereich
- 682: Dichtlippe
- 684: Handschutz
- 70: Kunststoff-Verbundbauteil
- 72: Kunststoff-Außenschicht
- 74: Elastomerschicht
- 75: Gewebe/ Gewirk/ Faserschicht
- 80: Kunststoff-Verbundbauteil
- 82: Glasfasergewebe
- 84: Elastomerschicht
- 85: Elastomer-Abschnitt
- 86: Carbon-Prepreg
- 88: Glas-Prepreg
- 90: Klebstofffilm
- 100: Kunststoff-Verbundbauteil
- 104: Elastomerschicht
- 106: Carbon-Prepreg

- F: (Senkrechte) Schwingungsenergie
- F_{S}: (horizontale) Scherkräfte
- F₁: Kraft
- A₁: (erste) Auslenkung
- A₂: (zweite) Auslenkung
- S: Schwenkbewegung
- V: Vertikaler Versatz
- H: Horizontale Verschiebung
- T: Torsion

## Patentansprüche

1. Kunststoff-Verbundbauteil (10) aus wenigstens zwei Schichten (12, 14, 16), wobei eine erste, zumindest teilweise aus einem wärmehärtenden Kunstharz bestehende Schicht (12) und eine zweite, zumindest teilweise aus einem Elastomer bestehende Schicht (14) durch einen Energieeintrag, wie eine gemeinsame Wärmebehandlung oder eine Bestrahlung mit UV-Licht, in einem Arbeitsgang zusammengefügt sind, und wobei die Elastomerschicht (14) wenigstens 0,5 pph (parts per hundred/ Anteile des Vernetzers pro hundert Anteile Gummi) wenigstens eines Vernetzers aus der Gruppe der Peroxide, der Amine und/oder der Bisphenole enthält, **dadurch gekennzeichnet, dass** das Kunststoff-Verbundbauteil (10) von einer dünnen harten Kunststoff-Außenschicht (12), wenigstens einer daran nach innen anschließenden Elastomerschicht (14) und wenigstens einer daran nach innen anschließenden Metall- und/oder Kunststoff-Trägerschicht (16) aus einem Faserverstärkten Kunststoff (FVK), einem Carbonfaserverstärkten Kunststoff (CFK) oder einem Glasfaserverstärkten Kunststoff (GFK) gebildet wird und als Splitter-Schutzteil oder als Schutzteil gegen ein plötzliches Totalversagen
• an einem Innen-Verkleidungsteil eines Fahrzeugs, wie einer Tür-Verkleidung, oder
• an einem äußeren Karosseriebauteil, wie einem Spoiler, einem Kotflügel, einem Fahrzeugdach, einer Heckklappe, einer Motorhaube (50), einer Crash-Nase oder einem Seitenteil eines Rennwagens, oder
• einem Rotorblatt, einer Tragfläche eines Sportflugzeugs, oder
• einer Prothese oder Orthese, oder
• an einem Rahmen, einem Lenker (40), einem Lenker-Vorbau oder einer Sattelstütze eines Zweirads, einem Tennisschläger, einer Schiene oder einer Fersenkappe von Inline-Skates, einem Body-Protektor, einem Schutzhelm, oder
• einem Verstärkungsteil eines gepanzerten Fahrzeugs, oder
• einem Splitterschutzteil oder Stichschutzteil als Schutz vor Schuss- oder Stichverletzungen
angeordnet ist oder dieses zumindest teilweise bildet.

2. Kunststoff-Verbundbauteil (10) nach Anspruch 1, **dadurch gekennzeichnet, dass** das Kunststoff-Verbundbauteil (10) als Aufprall-Schutzteil
• an der Vorderkante eines Flügels, einer Tragfläche oder eines Leitwerks eines Luftfahrzeugs, oder
• an der in Drehrichtung liegenden Vorderkante eines Rotorblattes (20) eines Hubschraubers oder eines Windrades, oder
• an einem Karosseriebauteil eines Fahrzeugs, wie einer Stoßstange oder einer Motorhaube (50), oder
• an einem aufwirbelnden Gegenständen ausgesetzten Bauteil eines Fahrzeugs, wie einem Unterbodenschutzteil eines Landfahrzeugs oder Schienenfahrzeugs, einer Fahrwerksstrebe, einem Lenkgestänge, einer Antriebswelle oder Kardanwelle, einem Tretlager eines insbesondere mit einem Carbon-Rahmen versehenen Mountain-Bikes, oder
• an einer Innenverkleidung eines Land-, Wasser-, Luft- oder Raum-Fahrzeugs, oder
• an der Innenseite eines zu Wartungszwecken begehbaren Hohlraums von Land-, Wasser-, Luft- oder Raum-Fahrzeugen zum Schutz vor Beschädigungen durch herunterfallende Werkzeuge, oder
• an den der Ladung zugewandten Oberflächen von offenen oder geschlossenen Transporträumen, wie Laderäumen von Transportern und Lastkraftwagen oder Containern, oder
• am vorderen Rumpfbereich oder Kielbereich eines Wasserfahrzeugs, wie eines Sportbootes, eines Speed-Bootes oder eines Kajaks, oder
• an stark beanspruchten Nutzflächen von Sportgeräten, wie dem Schaufelbereich von Eishockey-Schlägern, den Boden-Kontaktflächen von Nordic-Walking-Stöcken oder Ski-Stöcken oder den Paddelblättern von Kanu- oder Kajakpaddeln
angeordnet ist oder dieses zumindest teilweise bildet.

3. Kunststoff-Verbundbauteil (10) nach Anspruch 1, **dadurch gekennzeichnet, dass** das Kunststoff-Verbundbauteil (10) als Schutzteil gegen Vibrationen und Vibrationsschäden, gegen Resonanzen, zur Schwingungsdämpfung oder zur akustischen Dämpfung
• an einem Innen-Verkleidungsteil eines Fahrzeugs, wie einer Tür-Verkleidung, oder
• an einem äußeren Karosseriebauteil eines Fahrzeugs, wie einer Stoßstange, einem Fahrzeugdach, einer Fahrzeugtür, einem Kofferraumdeckel oder einer Motorhaube (50), oder
• an Schwingungen ausgesetzten Bauteilen im Maschinenbau, wie einem Roboterarm eines Industrieroboters, einem Griffbereich eines Pneumatikwerkzeugs, einem Rahmen, Gestell oder Gerüst, oder
• an Turbinen, Rotorblättern (20) oder Flügeln, oder
• an Messgeräten oder an optischen Vorrichtungen, oder
• an Baumaschinen, oder
• an Skiern oder Snowboards, oder
• an Brücken
angeordnet ist oder dieses zumindest teilweise bildet.

4. Kunststoff-Verbundbauteil nach einem der vorhergehenden Ansprüche, **dadurch gekennzeichnet, dass** die Kunststoff-Außenschicht (12) aus einem bereits fertig mit Kunstharz getränkten Gewebematerial (Prepreg) gebildet oder im Harz-Infusionsverfahren hergestellt ist.

5. Kunststoff-Verbundbauteil nach einem der vorhergehenden Ansprüche, **dadurch gekennzeichnet, dass** Kunststoff-Außenschicht (12) aus einem faserverstärkten Verbundkunststoff (FVK, CFK, GFK) oder aus Polyethylen (PE), insbesondere Ultra High Molecular Weight Polyethylen (UHMW-PE), High Molecular Weight Polyethylen (HMW-PE) oder Polytetrafluorethylen (PTFE), gebildet ist.

6. Kunststoff-Verbundbauteil nach einem der vorhergehenden Ansprüche, **dadurch gekennzeichnet, dass** die wenigstens eine Elastomerschicht (14) aus Ethylen-Propylen-Kautschuk (EPM), Ethylen-Propylen-Dien-Kautschuk (EPDM), Ethylen-Acrylat-Kautschuk (EAM), Fluorkarbon-Kautschuk (FKM), Acrylat-Kautschuk (ACM), Acrylnitril-Butadien-Kautschuk (NBR) optional im Verschnitt mit Polyvinylchlorid (PVC), Hydriertem Nitril-Kautschuk (HNBR), Carboxylat-Nitril-Kautschuk ((XNBR), Hydriertem Carboxylat-Nitril-Kautschuk (XHNBR), Naturkautschuk (NR), Ethyl-Vinyl-Acetat (EVA), Chlorsulfonyl-Polyäthylen-Kautschuk (CSM), Chloriertem Polyethylen (CM), Butyl-Kautschuk (BIIR) oder Halobutyl-Kautschuk, Silikon-Kautschuk (VMQ, MVQ), Fluor-Silikon-Kautschuk (FVMQ, MFQ), Chlorhydrin-Kautschuk (CO), Epichlorhydrin-Kautschuk (ECO), Polychloropren-Kautschuk (CR), einkomponentigem Polyurethan (PU) oder einer Kombination oder einem Verschnitt der vorstehend genannten Stoffe besteht, wobei 0,5 bis 15 pph, insbesondere 1,5 bis 5 pph eines Peroxids als Vernetzer vorgesehen sind.

7. Kunststoff-Verbundbauteil nach Anspruch 6, **dadurch gekennzeichnet, dass** die wenigstens eine Elastomerschicht (14) aus Ethylen-Propylen-Kautschuk (EPM), Ethylen-Propylen-Dien-Kautschuk (EPDM), Ethylen-Acrylat-Kautschuk (EAM), Fluorkarbon-Kautschuk (FKM), Acrylat-Kautschuk (ACM), Acrylnitril-Butadien-Kautschuk (NBR) optional im Verschnitt mit Polyvinylchlorid (PVC), Hydriertem Nitril-Kautschuk (HNBR), Carboxylat-Nitril-Kautschuk ((XNBR), Hydriertem Carboxylat-Nitril-Kautschuk (XHNBR), Naturkautschuk (NR), Ethyl-Vinyl-Acetat (EVA), Chlorsulfonyl-Polyäthylen-Kautschuk (CSM), Silikon-Kautschuk (VMQ, MVQ), Fluor-Silikon-Kautschuk (FVMQ, MFQ), einkomponentigem Polyurethan (PU) oder einer Kombination oder einem Verschnitt der vorstehend genannten Stoffe besteht, wobei 0,5 bis 25 pph, insbesondere 1,5 bis 5 pph eines wärmehärtenden Harzes auf Acrylatbasis als Ergänzung zur Unterstützung des Vernetzers vorgesehen sind.

8. Kunststoff-Verbundbauteil nach einem der Ansprüche 1 bis 5, **dadurch gekennzeichnet, dass** die wenigstens eine Elastomerschicht (14) aus Ethylen-Acrylat-Kautschuk (EAM), Fluorkarbon-Kautschuk (FKM), Acrylat-Kautschuk (ACM), Ethyl-Vinyl-Acetat (EVA), Chlorsulfonyl-PolyethylenKautschuk (CSM), Chloriertes Polyethylen (CM), Chlorhydrin-Kautschuk (CO), Epichlorhydrin-Kautschuk (ECO), Polychloropren-Kautschuk (CR) oder einer Kombination oder einem Verschnitt der vorstehend genannten Stoffe besteht und 0,5 bis 15 pph, insbesondere 1,5 bis 5 pph eines Amins als Vernetzer vorgesehen sind.

9. Kunststoff-Verbundbauteil nach einem der Ansprüche 1 bis 5, **dadurch gekennzeichnet, dass** die wenigstens eine Elastomerschicht aus Fluorkarbon-Kautschuk (FKM) besteht und 0,5 bis 15 pph, insbesondere 1,5 bis 5 pph eines Bisphenols als Vernetzer vorgesehen sind.

10. Kunststoff-Verbundbauteil nach einem der Ansprüche 1 bis 5, **dadurch gekennzeichnet, dass** die wenigstens eine Elastomerschicht (14) aus Chlorhydrin-Kautschuk (CO), Epichlorhydrin-Kautschuk (ECO), Polychloropren-Kautschuk (CR) oder einer Kombination oder einem Verschnitt der vorstehend genannten Stoffe besteht und 0,5 bis 15 pph, insbesondere 1,5 bis 5 pph eines Thioharnstoffs, eines Thioharnstoffderivates oder eines Dithiocarbonatderivats als Vernetzer vorgesehen sind.

11. Kunststoff-Verbundbauteil nach einem der Ansprüche 1 bis 10, **dadurch gekennzeichnet, dass** die wenigstens eine Elastomerschicht (14) aus Ethylen-Propylen-Kautschuk (EPM), Ethylen-Propylen-Dien-Kautschuk (EPDM), Ethylen-Acrylat-Kautschuk (EAM), Fluorkarbon-Kautschuk (FKM), Acrylat-Kautschuk (ACM), Acrylnitril-Butadien-Kautschuk (NBR) optional im Verschnitt mit Polyvinylchlorid (PVC), Hydriertem Nitril-Kautschuk (HNBR), Carboxylat-Nitril-Kautschuk ((XNBR), Hydriertem Carboxylat-Nitril-Kautschuk (XHNBR), Naturkautschuk (NR), Ethyl-Vinyl-Acetat (EVA), Chlorsulfonyl-Polyäthylen-Kautschuk (CSM), Silikon-Kautschuk (VMQ, MVQ), einkomponentigem Polyurethan (PU) oder einer Kombination oder einem Verschnitt der vorstehend genannten Stoffe besteht und 0,5 bis 15 pph, insbesondere 1,5 bis 5 pph eines Phenolharzes als Ergänzung zur Unterstützung des Vernetzers vorgesehen sind.

12. Kunststoff-Verbundbauteil nach einem der Ansprüche 1 bis 5, **dadurch gekennzeichnet, dass** die wenigstens eine Elastomerschicht (14) aus einem thermoplastischen Elastomer (TPE), wie Styrol/Ethenbuten/Styrol-Block-Copolymerisat (SEBS) oder Styrol/Butadien/Styrol-Block-Copolymerisat (SBS) oder einem thermoplastischen Elastomer auf der Basis von Polyurethan (TPU) oder einem Polyethylen niedriger Dichte (LDPE) oder Styrol/Butadien-Kautschuk (SBR) mit einem Styrolanteil von mehr als 50 % besteht.

13. Kunststoff-Verbundbauteil nach einem der vorhergehenden Ansprüche, **dadurch gekennzeichnet, dass** in die wenigstens eine Elastomerschicht (14) wenigstens ein Gewebe oder ein Gewirk oder eine Faserstruktur (18) derart eingebettet ist, dass dessen Fasern zumindest in Teilbereichen der Fläche vollständig vom Elastomer umgeben sind.

14. Kunststoff-Verbundbauteil nach Anspruch 13, **dadurch gekennzeichnet, dass** das Gewebe oder das Gewirk oder die Faserstruktur (18) aus einer Hochleistungsfaser wie Aramid oder Vectran® bestehen.
